Europäisches Patentamt

European Patent Office

Office européen des brevets

(19)

(11) Numéro de publication : **0 089 271**
**B1**

(12)                    FASCICULE DE BREVET EUROPÉEN

(45) Date de publication du fascicule du brevet :
28.05.86

(21) Numéro de dépôt : 83400460.8

(22) Date de dépôt : 07.03.83

(51) Int. Cl.⁴ : **A 61 F 13/20**, A 61 F 11/00

(54) **Perfectionnements à la fabrication des cotons-tiges et à leur procédé de fabrication.**

(30) Priorité : 12.03.82 FR 8204167
          13.08.82 FR 8214139

(43) Date de publication de la demande :
21.09.83 Bulletin 83/38

(45) Mention de la délivrance du brevet :
28.05.86 Bulletin 86/22

(84) Etats contractants désignés :
**CH DE FR GB IT LI NL**

(56) Documents cités :
     **DE-C-    487 198**
     **DE-C-    551 714**
     **DE-C-    580 729**
     **DE-C-    604 145**
     **FR-A- 2 249 640**
     **GB-A- 1 060 288**
     **US-A- 2 981 127**

(73) Titulaire : **Tran Dinh, Can**
**20 Avenue de Friedland**
**F-75008 Paris (FR)**

(72) Inventeur : **Tran Dinh, Can**
**20 Avenue de Friedland**
**F-75008 Paris (FR)**

(74) Mandataire : **Lerner, François**
**5, rue Jules Lefebvre**
**F-75009 Paris (FR)**

Jouve, 18, rue St-Denis, 75001 Paris, France

## Description

La présente invention concerne des perfectionnements à la fabrication des articles d'hygiène facilitant le nettoyage des oreilles, ces articles étant dénommés couramment « cotons-tiges ».

Les cotons-tiges ont pris, ces récentes années, un développement commercial important. Ils remplacent en effet avantageusement l'allumette ou l'épingle à cheveux autour de laquelle on enroulait autrefois un petit morceau de coton en vue d'effectuer le nettoyage de l'oreille et en particulier de son conduit auditif.

Cependant, ces cotons-tiges ne sont pas toujours bien supportés par les utilisateurs, et notamment ceux dont le conduit auditif est relativement restreint ou délicat. En particulier, l'utilisation des « cotons-tiges » est en général fortement déconseillée par les pédiatres et les médecins traitants chez les petits enfants.

Un emploi inadéquat d'un coton-tige peut provoquer en effet le refoulement du cérumen au fond du conduit auditif qui formera un bouchon de cérumen dont l'enlèvement devra être réalisé en milieu hospitalier. Bien évidemment, l'allumette portant à son extrémité une boule de coton enroulée sera sujette aux mêmes inconvénients, le pis-aller proposé étant d'utiliser un coton imbibé d'une solution alcoolique sans support rigide et dont l'efficacité est évidemment plus qu'aléatoire.

L'invention a pour objet de proposer un nouveau coton-tige permettant de résoudre les difficultés mentionnées. L'invention concerne également le procédé de fabrication de tels nouveaux cotons-tiges.

Conformément à l'invention, les difficultés mentionnées ci-dessus sont résolues par le fait que l'on propose un coton-tige comportant une tige à une extrémité au moins de laquelle est disposée une boule de coton ou analogue, le nouveau coton-tige selon l'invention se caractérisant en ce que sur la boule de coton ou analogue sont ménagées des saignées ou cannelures, la boule présentant en coupe transversale à l'axe de la tige une section non circulaire permettant l'introduction de la boule dans le conduit auditif sensiblement sans refoulement de cérumen au fond du conduit et la collecte du cérumen dans le volume des saignées ou cannelures.

Par exemple, selon un mode de réalisation, la section de la boule de coton sera généralement cruciforme, par exemple à quatre branches entre lesquelles sont formées lesdites saignées ou cannelures.

Selon une autre réalisation également très efficace, la boule à une forme générale sensiblement hélicoïdale avec saignées et cannelures en hélice.

On comprend immédiatement qu'en utilisant un tel coton-tige, après introduction du coton-tige dans le conduit auditif, et lorsqu'on tourne le coton-tige, le cérumen est recueilli dans les saignées ou cannelures (hélicoïdales ou longitudinales) ménagées entre les parties faisant protubérance ou saillie de la boule de coton, le risque de refoulement du cérumen par la boule étant considérablement restreint, et en pratique éliminé, du fait que le coton-tige lors de son introduction ne viendra porter que très localement contre la paroi du conduit auditif.

Un perfectionnement complémentaire à de tels « cotons-tiges » permet en outre d'éviter des accidents d'emplois plus particulièrement chez de jeunes enfants.

Chez les enfants un nettoyage fréquent des oreilles est nécessaire du fait de l'abondante production de cérumen.

Ce nettoyage, chez les tout petits, ne doit se faire que dans la partie visible : pavillons et parties externes du conduit auditif externe. Quelquefois le nettoyage peut se révéler très dangereux, du fait de la non coopération de l'enfant qui se débat et à cause de la zone réflexogène qui peut entraîner des gestes brusques.

Tous ces mouvements intempestifs font courir le risque, lors du nettoyage de l'oreille avec un « coton-tige », d'enfoncement brutal du « coton-tige » dans le conduit auditif externe avec possibilité de perforation traumatique du tympan par la tige relativement rigide support de la boule de coton.

Pour éviter ce risque, les « cotons-tiges » comportent un bouclier qui se fixe sur la tige sensiblement perpendiculairement à elle à une distance de son extrémité sur laquelle est fixée la boule de coton sensiblement inférieure à celle séparant l'entrée du conduit auditif au tympan du sujet auquel est destiné l'usage du « coton-tige ».

Avantageusement le bouclier se fixe de façon amovible à l'endroit précis déterminé de la tige, ledit bouclier et ladite tige comportant des moyens de butée et verrouillage coopérants.

Ainsi, sans augmentation sensible du prix de revient du « coton-tige », on éliminera radicalement les dangers réels que constituent les nettoyages fréquents nécessaires à une bonne hygiène des oreilles en particulier chez les jeunes enfants.

L'invention vise en outre un procédé permettant la fabrication pratique et automatisée de tels cotons-tiges perfectionnés.

L'invention, ses objets et avantages apparaîtront plus clairement à l'aide de la description qui va suivre faite en référence aux dessins annexés dans lesquels :

la figure 1 montre de façon schématique en coupe axiale un bâtonnet classique engagé dans le conduit auditif — et la manière dont il agit sur le cérumen ;

la figure 2 est une vue en coupe transversale faite selon le plan II-II de la figure 1 ;

la figure 3 est une vue semblable à celle de la figure 1 mais relative à un bâtonnet ou coton-tige conforme à l'invention ;

la figure 4 est une vue en coupe faite selon le plan IV-IV de la figure 3 ;

la figure 5 montre, comme la figure 3, un autre coton-tige conforme à l'invention et la manière dont il travaille ;

la figure 6 est une vue en coupe faite selon le plan VI-VI de la figure 5 ;

la figure 7 montre schématiquement en élévation un « coton-tige » conforme à l'invention pourvu des perfectionnements empêchant une trop grande introduction du bâtonnet ;

la figure 8 est une vue en coupe faite selon le plan VIII de la figure 7 ;

la figure 9 est une vue en élévation du bouclier séparé du « coton-tige », cette vue étant faite selon la flèche IX de la figure 10 ;

la figure 10 est une vue du côté du bouclier montré en élévation à la figure 9.

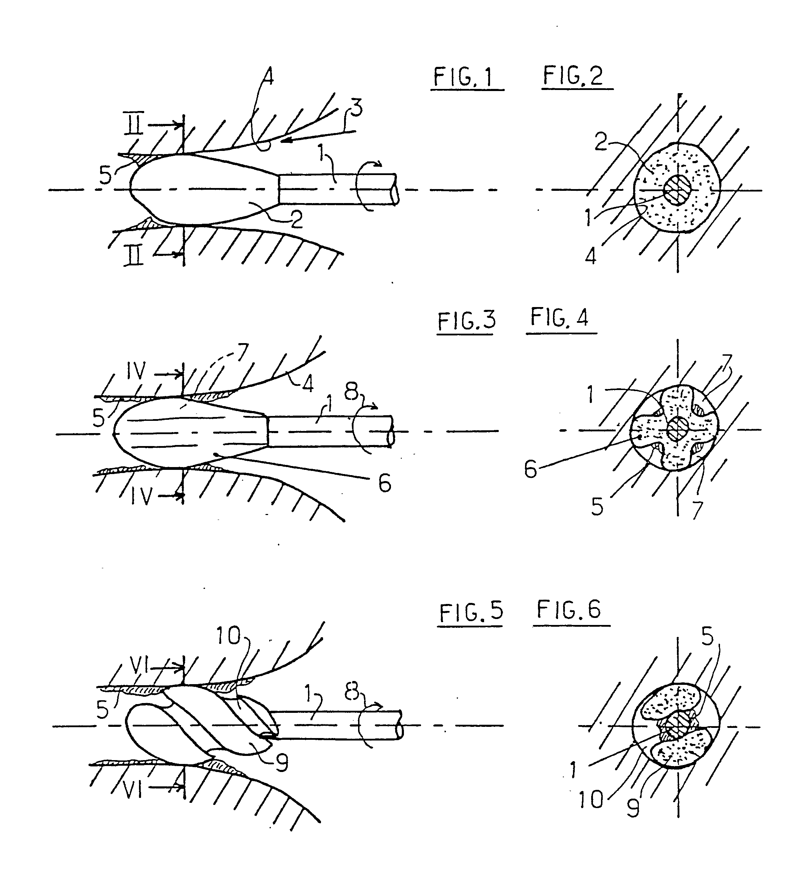

On se reportera tout d'abord aux figures 1 et 2 illustrant un bâtonnet formant coton-tige classique et son utilisation. Le coton-tige comprend la tige proprement dite 1 à une extrémité au moins de laquelle est enroulée une boule de coton ou analogue 2. Lorsqu'on veut nettoyer le conduit auditif, il faut introduire, comme indiqué par la flèche 3, l'extrémité du coton-tige portant la boule ovoïde 2 à l'intérieur du conduit auditif schématisé en 4. En opérant de cette façon, si le conduit auditif n'est pas très large, et en particulier chez les jeunes enfants, on repousse devant soi le cérumen 5, qui aura donc tendance à venir s'accumuler au fond du conduit auditif et à former un bouchon de cérumen qu'il sera ensuite très difficile d'extraire. Cet inconvénient s'explique immédiatement à la vue des figures 1 et 2, étant donné que le cérumen n'a pratiquement pas d'espace pour se rassembler sur la boule de coton 2.

Selon le mode de réalisation conforme à l'invention illustré aux figures 3 et 4, la boule de coton 6 a une forme générale sensiblement ovoïde comme la boule de coton 2 de l'art antérieur. Cependant, sa section n'est pas circulaire, mais comme il apparaît plus clairement à la figure 4, elle est cruciforme. Dans l'exemple illustré, la croix que forme la boule 6 a quatre branches entre lesquelles sont ménagées quatre saignées ou cannelures sensiblement longitudinales 7. Ces cannelures peuvent être éventuellement légèrement formées en hélice, ce qui améliorera l'efficacité du dispositif.

Il apparaît immédiatement qu'avec une telle conformation du coton le cérumen 5 ne sera pas repoussé au fond du conduit auditif mais pourra fluer dans les saignées ou cannelures 7 comme il apparaît clairement à la figure 4.

En outre, il apparaît clairement que dans ces conditions, un mouvement de rotation du bâtonnet sur lui-même comme indiqué par la flèche 8 aura tendance à récolter tout le cérumen le long du conduit auditif à l'intérieur des saignées ou cannelures 7.

Selon la réalisation illustrée aux figures 5 et 6, la boule de coton 9 est formée avec une cannelure ou saignée hélicoïdale 10. Pour les mêmes raisons qu'expliquées ci-dessus en relation avec les figures 3 et 4, le cérumen 5 n'aura dans ces conditions que peu tendance à être refoulé vers le fond du conduit auditif, fluant et s'accumulant dans la cannelure 10 (ou les cannelures 10 si plusieurs cannelures hélicoïdales parallèles sont formées sur la boule de coton 9). En outre, par un mouvement de rotation comme indiqué par la flèche 8 du bâtonnet 1, on aura tendance à extraire le cérumen hors du conduit.

De façon à former la boule du coton-tige, telle par exemple que la boule 6 ou la boule 9 des exemples illustrés, on pourra procéder de diverses manières.

Par exemple, selon un procédé, on pourra former le coton-tige sensiblement comme selon les techniques antérieures. Ensuite, il suffira de venir comprimer, de préférence à chaud dans un outil de forme convenablement appropriée la boule de coton pour la « repasser » dans la forme souhaitée.

Cette opération peut se faire rapidement, facilement et économiquement.

Si l'on veut améliorer la tenue de la conformation de la boule « rainurée », on pourra revêtir la boule de coton d'un apprêt convenable, tel par exemple que de l'amidon.

D'autres procédés de fabrication peuvent cependant être employés.

Par exemple, pour former une boule de coton telle qu'illustrée au mode de réalisation des figures 5 et 6, on pourra enrouler en hélice sur l'extrémité du bâtonnet un brin de coton. Eventuellement, une colle pourra être appliquée sur le bout du bâtonnet pour faciliter l'enroulement et la bonne tenue du brin de coton.

Eventuellement, en lieu et place du coton, on pourra utiliser d'autres matières, telles par exemple que des mousses synthétiques qui pourront être facilement thermoformées avec les sections convenables, à condition de prendre des matières non allergisantes en soi connues et de souplesse appropriée.

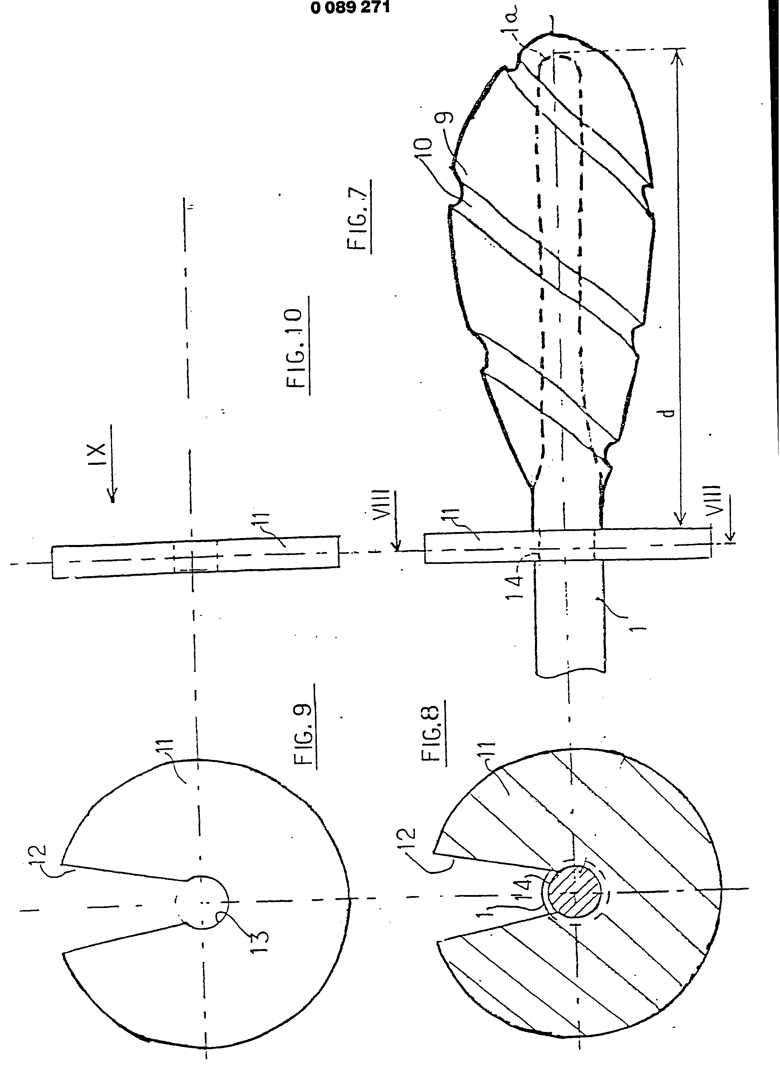

Selon les perfectionnements illustrés aux figures 7 à 10 des dessins, on retrouve la tige support 1 du coton-tige comportant à l'une de ses extrémités au moins une boule de coton 9 formée avec une cannelure hélicoïdale 10 facilitant l'opération de nettoyage des oreilles en évitant le refoulement de cérumen au fond du conduit auditif.

En outre, on a fixé vers l'extrémité du coton-tige comportant la boule de coton 9 un bouclier 11 amovible sensiblement circulaire plat présentant une encoche 12 se terminant en son centre par un orifice 13 dont le diamètre correspond sensiblement à celui d'une saignée ou collerette 14 ménagée autour de la tige 1 comme il apparaît plus clairement à la figure 1. La distance d séparant le bouclier 11 de l'extrémité libre 1a de la tige 1 est choisi de façon à être généralement sensiblement inférieure à celle séparant l'entrée du conduit auditif au tympan du sujet auquel est destiné l'usage du « coton-tige », par exemple un jeune enfant de 2 à 5 ans. Une marge de sécurité raisonnable peut être prise, dans la mesure où, comme mentionné précédemment, il n'est pas nécessaire de nettoyer le conduit auditif jusqu'au fond.

Le bouclier 11 est avantageusement constitué en une matière plastique transparente ou translucide souple qui vient se verrouiller élastiquement sur la saignée 14 par simple enclenchement sur la tige. De cette façon, même en cas de mouvements intempestifs de l'enfant, on ne risque pas d'accidents, le bouclier empêchant toute pénétration du « coton-tige » au-delà de la distance d autorisée.

L'avantage de prévoir un bouclier amovible est qu'il suffit d'un seul bouclier pour une boîte de « coton-tige », et en outre le bouclier pouvant être ainsi séparé n'entraine aucun problème de stockage et de conditionnement de l'article.

Bien entendu d'autres moyens de fixation et de mise en place du bouclier peuvent être prévus sans sortir du cadre des perfectionnements de l'invention. En outre, deux ou un plus grand nombre de saignées peuvent être prévues plus ou moins rapprochées de l'extrémité 1a de la tige, la saignée convenable pouvant être choisie en fonction par exemple de l'âge de l'enfant auquel est destiné l'usage du « coton-tige ».

## Revendications

1. Coton-tige, destiné plus particulièrement au nettoyage des oreilles, comportant une tige (1) à une extrémité au moins de laquelle est disposée une boule de coton ou analogue (6, 9), caractérisé en ce que sur la boule de coton ou analogue (6, 9) sont ménagées des saignées ou cannelures (7, 10), la boule présentant en coupe transversale à l'axe de la tige (1) une section non circulaire permettant l'introduction de la boule dans le conduit auditif sensiblement sans refoulement de cérumen au fond du conduit et la collecte du cérumen dans le volume des saignées ou cannelures (7, 10).

2. Coton-tige selon la revendication 1, caractérisé en ce que ladite section de la boule (6) est généralement cruciforme.

3. Coton-tige selon la revendication 1, caractérisé en ce que ladite boule (9) a une forme sensiblement hélicoïdale.

4. Coton-tige selon l'une des revendications précédentes, caractérisé en ce que ladite boule (6, 9) est inscrite sensiblement dans une forme ovoïde.

5. Coton-tige selon l'une des revendications précédentes caractérisé en ce qu'il comporte un bouclier (11) qui se fixe sur la tige (1) sensiblement perpendiculairement à elle à une distance d de son extrémité (1a) sur laquelle est fixée la boule de coton, cette distance étant sensiblement inférieure à celle séparant l'entrée du conduit auditif au tympan du sujet auquel est destiné l'usage du coton-tige.

6. Coton-tige selon la revendication 5 caractérisé en ce que ledit bouclier (11) se fixe de façon amovible à l'endroit précis déterminé de la tige.

7. Coton-tige selon la revendication 5 ou la revendication 6 caractérisé en ce que ledit bouclier (11) et ladite tige (1) comportent des moyens de butée et de verrouillage (13, 14) coopérants.

8. Coton-tige selon l'une des revendications 5 à 7 caractérisé en ce que ledit bouclier (11) se présente sous la forme d'une plaquette dans laquelle est ménagée une fente, échancrure ou analogue (12) qui vient s'adapter sur une saignée ou collerette (14) formée sur la tige (1).

9. Coton-tige selon l'une des revendications 5 à 8 caractérisé en ce que ledit bouclier est formé d'une matière plastique transparente ou translucide.

10. Procédé de fabrication de cotons-tiges selon l'une des revendications précédentes, caractérisé en ce que la boule de coton (6, 9) ou analogue est formée à chaud par compression.

11. Procédé de fabrication de cotons-tiges selon l'une des revendications 1 à 9, caractérisé en ce que la boule de coton ou analogue est fixée à la tige par collage.

12. Procédé de fabrication de cotons-tiges selon la revendication 3, caractérisé en ce que la boule de coton est formée par enroulement en hélice d'un brin de coton sur ladite tige.

13. Procédé de fabrication de cotons-tiges selon l'une des revendications 10 à 12 caractérisé en ce que la boule de coton (6, 9) est fixée par un apprêt, tel par exemple que de l'amidon.

## Claims

1. Cotton swab, more particularly for cleaning the ears, comprising a rod (1), at at least one end of which is disposed a ball of cotton or the like (6, 9), characterised in that on the ball of cotton or the like (6, 9) are provided grooves or channels (7, 10), the ball having, transverse to the axis of the rod (1), a non-circular cross-section which permits both the introduction of the ball into the auditory passage substantially without causing an accumulation of wax at the end of the passage, and the collection of wax in the space formed by the grooves or channels (7, 10).

2. Cotton swab according to claim 1, characterised in that the said cross-section of the ball (6) is generally cruciform.

3. Cotton swab according to claim 1, characterised in that the said ball (9) has a substantially helicoidal form.

4. Cotton swab according to any one of the preceding claims, characterised in that the said ball (6, 9) is inscribed substantially within an ovoidal form.

5. Cotton swab according to any one of the preceding claims, characterised in that it includes a shield (11) fixed to the rod (1) substantially perpendicular thereto at a distance d from its end (1a) to which is fixed the cotton ball, this distance being substantially less than that separating the entrance of the auditory passage and the ear drum of the subject for whom use of the cotton swab is intended.

6. Cotton swab according to claim 5, characterised in that the said shield (11) is removably fixed at the precisely determined position on the rod.

7. Cotton swab according to claim 5 or claim 6, characterised in that the said shield (11) and the said rod (1) include co-operating abutment and locking means (13, 14).

8. Cotton swab according to any one of claims 5 to 7, characterised in that the said shield (11) is in the form of a platelet within which is located a slot indentation, or the like (12) which fits onto a groove or flange (14) formed on the rod (1).

9. Cotton swab according to any one of claims 5 to 8 characterised in that the said shield is formed of a transparent or translucent plastics material.

10. Process for the manufacture of cotton swabs according to any of the preceding claims, characterised in that the cotton ball (6, 9) or the like is heat-formed under compression.

11. Process for the manufacture of cotton swabs according to any one of claims 1 to 9, characterised in that the ball of cotton or the like is fixed to the rod by glueing.

12. Process for the manufacture of cotton rods according to claim 3, characterised in that the cotton ball is formed by rolling a strand of cotton onto the rod to form a helix.

13. Process for the manufacture of cotton swabs according to any one of claims 10 to 12 characterised in that the ball of cotton (6, 9) is fixed by a size, such as for example starch.

## Patentansprüche

1. Wattestäbchen, insbesondere zur Reinigung von Ohren, mit einem Stäbchen (1), an dessen mindestens einem Ende eine Wattekugel oder dergleichen (6, 9) angeordnet ist, dadurch gekennzeichnet, daß auf der Wattekugel oder dergleichen (6, 9) Kerben oder Rillen (7, 10) angeordnet sind und die Kugel einen nicht kreisrunden Querschnitt quer zur Achse des Stäbchens (1) hat, wobei das Einführen der Kugel in den Gehörgang im wesentlichen ohne Zurückdrängen von Ohrenschmalz an den Grund des Ganges bzw. der Leitung und die Sammlung von Ohrenschmalz im Volumen der Kerben oder Rillen (7, 10) möglich ist.

2. Wattestäbchen nach Anspruch 1, dadurch gekennzeichnet, daß der Schnitt der Kugel (6) im allgemeinen Kreuzförmig ist.

3. Wattestäbchen nach Anspruch 1, dadurch gekennzeichnet, daß die Kugel im wesentlichen Schraubenform hat.

4. Wattestäbchen nach einem der vorhergehenden Ansprüche, dadurch gekennzeichnet, daß die Kugel (6, 9) im wesentlichen in einer eiförmigen Form eingeschrieben ist.

5. Wattestäbchen nach einem der vorhergehenden Ansprüche, dadurch gekennzeichnet, daß es eine Abschirmung (11) aufweist, die auf dem Stäbchen (1) im wesentlichen senkrecht zu diesem in einem Abstand (d) seines Endes (la) angebracht ist, an welchem die Wattekugel befestigt ist, wobei dieser Abstand im wesentlichen kleiner als derjenige ist, welcher den Eintritt des Gehörganges vom Trommelfell des Patienten trennt, für welches die Benutzung des Wattestäbchens bestimmt ist.

6. Wattestäbchen nach Anspruch 5, dadurch gekennzeichnet, daß die Abschirmung verstellbar an genau bestimmter Stelle des Stäbchens angebracht ist.

7. Wattestäbchen nach Anspruch 5 oder Anspruch 6, dadurch gekennzeichnet, daß die Abschirmung (11) und das Stäbchen (1) zusammenwirkende Anschlag- und Verriegelungsmittel (13, 14) aufweisen.

8. Wattestäbchen nach einem der Ansprüche 5 bis 7, dadurch gekennzeichnet, daß die Abschirmung (11) die Form eines Plättchens hat, in welchem ein Schlitz, eine Aussparung oder dergleichen (12) angeordnet ist, der bzw. die an eine Kerbe oder einen Bund (14), die bzw. der auf dem Stäbchen (1) gebildet ist, angepaßt ist.

9. Wattestäbchen nach einem der Ansprüche 5 bis 8, dadurch gekennzeichnet, daß die Abschirmung aus einem transparenten oder durchscheinenden Kunststoffmaterial hergestellt ist.

10. Verfahren zur Herstellung des Wattestäbchens nach einem der vorhergehenden Ansprüche, dadurch gekennzeichnet, daß die Wattekugel (6, 9) oder dergleichen warm durch Zusammendrückung gebildet wird.

11. Verfahren zur Herstellung von Wattestäbchen nach einem der Ansprüche 1 bis 9, dadurch gekennzeichnet, daß die Wattekugel oder dergleichen durch Klebung am Stäbchen befestigt wird.

12. Verfahren zum Herstellen von Wattestäbchen nach Anspruch 3, dadurch gekennzeichnet, daß die Wattekugel durch schraubenförmiges Aufwickeln eines Watte- bzw. Baumwollstranges auf dem Stäbchen gebildet wird.

13. Verfahren zum Herstellen von Wattestäbchen nach einem der Ansprüche 10 bis 12, dadurch gekennzeichnet, daß die Wattekugel (6, 9) durch eine Appretur, wie z. B. Stärke, fixiert wird.

FIG. 1 FIG. 2

FIG. 3 FIG. 4

FIG. 5 FIG. 6

FIG. 9

FIG. 10

FIG. 7

FIG. 8

0 089 271